# EUROPÄISCHE PATENTSCHRIFT

(11) **EP 1 409 493 B1**
(45) Veröffentlichungstag und Bekanntmachung des Hinweises auf die Patenterteilung: **15.04.2009**
(21) Anmeldenummer: 02735103.0
(22) Anmeldetag: 25.01.2002
(51) Int. Cl.: C07F 9/50, C07F 17/02, C07C 29/145

(54) **VERFAHREN ZUR HERSTELLUNG VON NICHT-CHIRALEN UND OPTISCH AKTIVEN HYDROXYGRUPPEN ENTHALTENDEN ORGANISCHEN VERBINDUNGEN**
METHOD FOR PRODUCING NON-CHIRAL ORGANIC COMPOUNDS CONTAINING OPTICALLY ACTIVE HYDROXY GROUPS
PROCEDE DE PREPARATION DE COMPOSES ORGANIQUES NON CHIRAUX CONTENANT DES GROUPES HYDROXY OPTIQUEMENT ACTIFS

(30) Priorität: 05.02.2001 DE 10105104
(43) Veröffentlichungstag der Anmeldung: 21.04.2004
(73) Patentinhaber: Arlt, Dieter, 31737 Rinteln (DE)
(72) Erfinder: Arlt, Dieter, 31737 Rinteln (DE)
(86) Internationale Anmeldenummer: PCT/EP2002/000808
(87) Internationale Veröffentlichungsnummer: WO 2002/076997

(56) Entgegenhaltungen:
- EP-A- 0 718 265
- US-A- 5 457 219
- US-A- 6 162 951
- TER HALLE R ET AL: "'Diam-BINAP';a highly efficient monomer for the synthesis of heterogeneous enantioselective catalysts" TETRAHEDRON LETTERS, ELSEVIER SCIENCE PUBLISHERS, AMSTERDAM, NL, Bd. 41, Nr. 5, Januar 2000 (2000-01), Seiten 643-646, XP004187745 ISSN: 0040-4039 in der Anmeldung erwähnt
- ABDUR-RASHID K ET AL: "RUHCL(DIPHOSPHINE)(DIAMINE): CATALYST PRECURSORS FOR THE STEREOSELECTIVE HYDROGENATION OF KETONES AND IMINES" ORGANOMETALLICS, ACS, COLUMBUS, OH, US, Bd. 20, Nr. 6, 19. März 2001 (2001-03-19), Seiten 1047-1049, XP001033320 ISSN: 0276-7333
- DOUCET H ET AL: "TRANS-ÄRUCI2(PHOSPHANE)2(1,2-DIAMINE)Ü AND CHIRAL TRANS-ÄRUCL2(DIPHOSPHANE)(1,2-DIAMINE): SHELF-STABLE PRECATALYSTS FOR THE RAPID, PRODUCTIVE, AND STEREOSELECTIVE HYDROGENATION OF KETONES" ANGEWANDTE CHEMIE. INTERNATIONAL EDITION, VERLAG CHEMIE. WEINHEIM, DE, Bd. 37, Nr. 12, 1998, Seiten 1703-1707, XP002938991 ISSN: 0570-0833

## Beschreibung

Hydroxygruppen enthaltende organische Verbindungen, auch in optisch aktiver Form, sind wichtige Zwischenprodukte, beispielsweise zur Herstellung von pharmazeutischen Wirkstoffen, Pflanzenschutzmitteln, Riechstoffen und flüssigkristallinen Substanzen.

Aus der EP-A 718 265 ist ein Verfahren zur Herstellung von nicht-chiralen und optisch aktiven Alkoholen bekannt, bei dem man eine Carbonylverbindung mit Wasserstoff in Gegenwart eines homogenen Katalysators, einer Base und einer Stickstoff enthaltenden organischen Verbindung umsetzt. Bei dem homogenen Katalysator kann es sich z.B. um einen Ruthenium-Komplex mit Phosphinliganden handeln, bei der Base um ein Alkali- oder Erdalkalimetallhydroxid und bei der Stickstoff enthaltenden organischen Verbindung um ein Amin.

Nachteilig bei diesem Verfahren ist der Einsatz eines homogenen Katalysators, was die Aufarbeitung des Reaktionsgemisches und die Herstellung von nicht mit Katalysatoren oder deren Bestandteilen verunreinigten Produkten erschwert. Außerdem ist die Wiedergewinnung des wertvollen Katalysators oder seiner Bestandteile, wenn überhaupt, nur mit hohem technischen und wirtschaftlichen Aufwand möglich. Schließlich ist es schwierig, Verfahren unter Verwendung von homogenen Katalysatoren kontinuierlich durchzuführen.

Homogene Katalysatoren zeichnen sich durch hohe Selektivitäten und Aktivitäten aus, die von entsprechenden heterogenen Katalysatoren i.a. nicht erreicht werden.

Es musste deshalb damit gerechnet werden, dass auch im vorliegenden Fall beim Übergang von homogenen zu heterogenen Katalysatoren eventuelle Vorteile, z.B. hinsichtlich Aufarbeitung des Reaktionsgemisches, Reinheit des hergestellten Produkts, Katalysatorrückgewinnung und kontinuierliche Reaktionsführung, nur verbunden mit gravierenden Nachteilen, z.B. hinsichtlich Selektivität und Aktivität, realisiert werden können.

Es wurde nun ein Verfahren zur Herstellung von nicht-chiralen und optischen aktiven Alkoholen gefunden, bei dem man eine Carbonylverbindung mit Wasserstoff in Gegenwart eines Katalysators, einer Base und gegebenenfalls eines Diamins umsetzt, das dadurch gekennzeichnet ist, dass man als Katalysator einen trägergebundenen Ru(II)- Biphosphin-Diamin-Ru-Komplex-Katalysator der Formel (I) einsetzt

[Träger]-[gegebenenfalls Modifizierung]-[Bindungsgruppe]--[Bisphosphin 〉RuHal₂〈 Diamin (I)

Hal = Cl oder Br

Vor kurzem (Synlett 2000, No. 5 680-682), ist ein Verfahren zur asymmetrischen Hydrierung von Ketonen bekannt geworden, das unter Verwendung einer heterogenen Katalysatorkomponente durchgeführt wird, die in der Hauptkette eingebaute BINAP-Strukturelemente enthält. Es handelt sich dabei um ein oligomeres Diisocyanataddukt mit der Bezeichnung "Poly-NAP" (siehe dazu Tetrahedron Letters 41 (2000), 643-646), das von den erfindungsgemäß verwendeten Katalysatoren, die trägergebundene Bisphosphin-Diamin-Ru(II)-Komplexe enthalten, deutlich unterschieden ist. Die erfindungsgemäß verwendeten, trägergebundenen Katalysatoren sind z.B. im Unterschied zu Poly-NAP in allen Lösungsmitteln unlöslich. Ein wesentlicher Vorteil des erfindungsgemäßen Verfahrens besteht darin, dass aufgrund der Vielfalt der chiralen Bisphosphine, die für den Aufbau trägergebundener Katalysatoren infrage kommen, eine Vielzahl von unterschiedlichen heterogenen Bisphosphin-Komponenten zur Verfügung gestellt werden kann, um in Kombination mit den Aminmit den Amin-Komponenten des Katalysatorsystems die optimale Verfahrensweise für das jeweilige Substrat zu erreichen.

Katalysatoren, die trägergebundene Bisphosphinliganden enthalten und die als Vorstufen für die erfindungsgemäß verwendeten, neuen Katalysatoren in Frage kommen, sind bekannt oder können analog zur Herstellung der bekannten erhalten werden (siehe z.B. J. Org. Chem. 63, 3137 (1998), GB-A 96-19684, EP-A 496 699, EP-A 496 700, EP-A 728 768, J. Mol. Catal. A 107 (1-3), 273 (1996) und 13^{th} International Conference on Org. Synth., Warsaw, July 1-5, 2000, Book of Abstracts, PB-4, S. 227).

Ein Verfahren zur Herstellung von nicht-chiralen Alkoholen unter Verwendung solcher Katalysatoren in Gegenwart von Aminen und einer Base ist bisher jedoch nicht in Betracht gezogen worden.

Erfindungsgemäß werden Alkohole durch Umsetzung einer Carbonylverbindung mit Wasserstoff in vorteilhafter Weise erhalten, wenn man die Hydrierung unter Verwendung eines Katalysators der Formel (I) in Gegenwart einer Base ausführt.

[Träger]-[gegebenenfalls Modifizierung]-[Bindungsgruppe]--[Bisphosphin 〉RuHal₂〈 Diamin] (I),

wobei
**Hal** Chlor oder Brom
bedeutet.

Es ist auch möglich, die Hydrierung unter Verwendung eines trägergebundenen, unlöslichen Katalysators der Formel (II) auszuführen, wenn während der Hydrierung gleichzeitig sowohl eine Base als auch ein Diamin im Reaktionsgemisch vorhanden sind. In diesem Falle wird ein Katalysator der Formel (I) in situ gebildet. 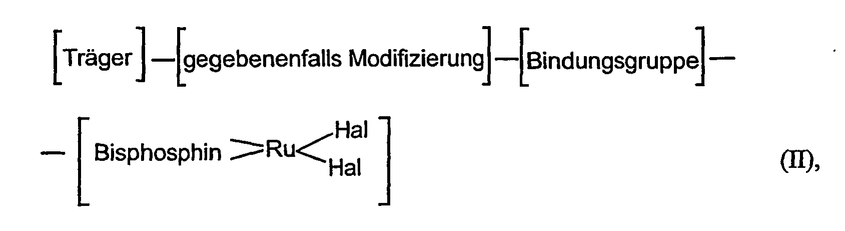 wobei
**Hal** Chlor oder Brom
bedeutet.

Bevorzugt werden jedoch erfindungsgemäß Katalysatoren der Formel (I) verwendet, die bereits trägergebundene Ru(II)-Komplexe enthalten, die jeweils sowohl Bisphosphin- als auch Diamin-Liganden enthalten.

Als Träger für die erfindungsgemäß einzusetzenden Katalysatoren kommen anorganische Materialen, z.B. Kieselgele, und organische Materialien, z.B. vernetzte Polymere, in Frage.

Als anorganische Träger seien beispielsweise genannt: Silicate oder Metalloxide in Pulverform mit einer durchschnittlichen Partikelgröße zwischen 10 nm und 2000 µm, vorzugsweise 10 nm und 500 µm. Die Partikel können sowohl kompakt als auch porös sein, wobei im letzteren Falle die innere Oberfläche zwischen 1 und 1200 m² liegt. Als Beispiele für oxidische Träger seien SiO₂, TiO₂, ZrO₂, MgO, WO₃, Al₂O₃, und La₂O₃ genannt, für Silicate Kieselgele, Tonerden, Zeolithe und poröses Glas (Controlled Pore Glass). Bevorzugte Träger sind Kieselgele und Aluminiumoxide.

Als organische Träger des Katalysators dienen beispielsweise vernetzte Perlpolymerisate, die durch Suspensionspolymerisation unter Zusatz von bifunktionellen Monomeren aus Styrol, Acryl- oder Methacrylsäureestern oder (Meth)acrylamiden erhalten werden können.

Um eine Anbindung der Bisphosphinliganden zu ermöglichen, müssen die Träger reaktive Gruppen enthalten. Dafür kommen z.B. primäre und sekundäre Aminogruppen, Hydroxyl-, Carboxyl- und Isocyanat-Gruppen in Betracht sowie reaktives Halogen enthaltende Gruppierungen wie benzylisches Chlor oder Brom(ar)alkyl.

Solche Gruppierungen können bereits bei der Herstellung des Perlpolymerisates durch Verwendung von funktionellen Comonomeren wie Acrylsäure, Methacrylsäure, Acrylsäure-(2-hydroxyethylester), Acrylsäure-(2-methyl-2-isocyanato-propylester) oder durch nachfolgende Modifizierung des Trägers, z.B. durch Chlormethylierung des vernetzten Polstyrolperlpolymerisates, an die sich gegebenenfalls eine weitere Funktionalisierung wie z.B. Verseifung und Polyether-Pfropfung anschließen kann. Die Herstellung solcher Polymerisate mit reaktiven Gruppen ist bekannt.

Es hat sich als vorteilhaft erwiesen, die Modifizierung des Trägers so zu gestalten, dass zwischen Träger und Bisphosphin ein größerer Abstand gehalten wird, vorteilhaft ist ein Abstandshalter, der aus einer Alkylen- oder Aralkylen- oder einer Alkylenoxy-Kette gegebenenfalls mit eingebauten Ester-, Ether-, Amid-, Urethan- oder Harnstoff-Gruppierungen besteht und mindestens 6, bevorzugt mindestens 12 Atome zwischen Träger und Bisphosphin umfasst.

Die anorganischen Träger - insbesondere Kieselgele - können durch Umsetzung mit Kieselsäureestern oder Chlorsilanen, die jeweils geeignete funktionelle Gruppen ent halten, in an sich bekannter Weise modifiziert werden, um für die gewünschte Verknüpfung geeignete Reaktivgruppen wie z.B. Aminogruppen einzuführen. Als Verbindungen, die für eine solche Modifizierung in Betracht kommen, seien beispielsweise genannt 3-Aminopropyl-triethoxysilan, Trichlorvinylsilan und 3-Mercaptopropyl-trimethoxysilan.

Es ist auch möglich den anorganischen Träger mit geeigneten modifizierten Bisphosphinderivaten direkt zu den erfindungsgemäßen, fixierten Bisphosphin-(derivaten) umzusetzen. Für diese Modifizierung werden Bisphosphinderivate eingesetzt, die funktionelle Gruppen der Formel

Si(OR)₃₋ₙ(R)ₙ

oder

Si(R')ₙCl₃₋ₙ

enthalten,
wobei
- R: für Alkyl,
- R': für Alkyl oder Alkoxy und
- n: für 0 - 2 stehen.

Die Umsetzung erfolgt analog zu bekannten Modifizierungen von Kieselgelen mit Chlorsilanen bzw. Kieselsäureestern.

Für die Herstellung der erfindungsgemäß eingesetzten Katalysatoren werden chelatbildende Bisphosphine verwendet, die funktionelle Gruppen enthalten, die eine kovalente Bindung mit reaktiven Gruppierungen an einem geeigneten oder geeignet modifizierten, unlöslichen Träger erzeugen können.

Als funktionelle Gruppen der Bisphosphinderivate, die zur Verknüpfung mit den reaktiven Gruppierungen der zuvor beschriebenen, gegebenenfalls entsprechend modifizierten Träger eingesetzt werden, seien beispielsweise genannt: aromatisch oder (ar)aliphatisch gebundene primäre oder sekundäre Aminogruppen, aromatisch oder (ar)aliphatisch gebundene Hydroxylgruppen, Carboxyl- und Isocyanat-Gruppen sowie aromatisch gebundene Chlormethyl- und Chlorsulfonyl-Gruppen.

Besonders geeignet sind (co)polymerisationsfähige Gruppierungen wie z.B. aromatische Vinylgruppen, (Meth)acrylsäureester- oder (Meth)acrylamid-Gruppen.

Die Verknüpfung kann sowohl mit entsprechend funktionalisierten Bisphosphinen als auch mit den analogen Bisphosphinoxiden ausgeführt werden. Bei Verwendung von Chlorsulfonyl- oder Chlormethylgruppen ist die Ausführung auf der Bisphosphinoxidstufe, um Nebenreaktionen zu vermeiden, obligatorisch.

Im Falle der Verknüpfung mit dem polymeren Träger auf der Phosphinoxid-Stufe ist es notwendig, nachfolgend das trägergebundene Bisphosphinoxid in an sich bekannter Weise mit Silanen in Gegenwart von tertiären Aminen zum polymergebundenen Bisphosphin zu reduzieren.

Dementsprechend werden beispielsweise bindungsfähige funktionelle Gruppen enthaltende Derivate von 1,2-Bis(diphenylphosphino-)ethan, 1,2- und 1,3-Bis(diphenylphosphino-)propan, (Phenylen-1,2-diyl)bis(diphenylphosphin), Pyrrolidin-3,4-diyl)-bis(diphenylphosphin) (unmodifiziert), und insbesondere zur Herstellung von enantioselektiv wirksamen Katalysatoren Derivate mit bindungsfähigen funktionellen Gruppen der chiral einheitlichen chelatbildenden Bisphosphine Dipamp, Prophos, Norphos, Chiraphos, Deguphos (unmodifiziert), Diop, ModDiop, Bppm, ModBppm, Duphos und BppfOH (unmodifiziert), verwendet (zu der Bedeutung der Abkürzungen siehe Handbook of Enantioselective Catalysis, Ed. H. Brunner, W. Zettlmeier, VCH Verlag Weinheim, 1993).

Besonders bevorzugt werden zur Verknüpfung geeignete Gruppen enthaltende Derivate atropisomerer Bisphosphine, insbesondere in chiral einheitlicher Form, als Bausteine für die erfindungsgemäßen Katalysatoren benutzt. Hier seien beispielsweise bindungsfähige funktionelle Gruppen enthaltende enantiomerenreine Derivate von 2,2'-Bis(diarylphosphino)-1,1'-binaphthylen wie 5,5'-Diamino-2,2'-bis(diphenylphosphino)-1,1-binaphthyl, 7,7'-Dihydroxy-2,2'-bis(di-(m-xylyl)phosphino(-1,1'-binaphthyl, 4-(2,2'-bis(diphenylphosphinol)-1,1'-binaphth-6-yl)butansäure, 4-(2,2'-bis(diphenylphosphinol)-1,1'-binaphth-6-yl)butanol oder zur Verknüpfung mit geeigneten Trägern befähigte Gruppen enthaltende Derivate von mindestens in 6,6'-Position substituierten (Biphenyl-2,2'-diyl)bis(diarylphosphinen), (Biphenyl-2,2'-diyl)bis(dicycloalkylphosphinen) oder Biphenyl-2,2'-diyl)bis(dihetarylphosphinen), wie beispielsweise (6,6'-Dihydroxybiphenyl-2,2'-diyl)bis(diphenylphosphin), (6-Hydroxy-6'-methoxybiphenyl-2,2'-diyl)bis(di-(m-xylyl)phosphin, (6,6'-Dihydroxy-biphenyl-2,2'-diyl)bis(dicyclohexylphosphin) und (6,6'-Dihydroxybiphenyl-2,2'-diyl)-bis(di-thien-2-ylphosphin) aufgeführt.

Man kombiniert nun gegebenenfalls modifiziertes Trägermaterial und modifizierte Phosphine so, dass beide Komponenten eine chemische Bindung untereinander eingehen können. Eine Komponente kann z.B. COOH-Gruppen und die andere Komponente NH₂-Gruppen enthalten, die unter der Entstehung von -CO-NH-Bindungen miteinander reagieren können. Je nach gewählter Kombination von reaktiven Gruppen können verschiedenartige Bindungen realisiert werden, z.B. außer -CO-NH- auch -CO-NR-, CO-O-, -O-, -OCONH-, -NH-CO-NH, -O-CO-NR- und -O-CO-O-. Die Methoden, entsprechend reaktive Stoffe an Träger zu koppeln, sind bekannt.

Ein weiterer Gegenstand der vorliegenden Erfindung Ru(II)-Komplex-Katalysator mit der Formel (I)

[Träger]-[gegebenenfalls Modifizierung]-[Bindungsgruppe]--[Bisphosphin 〉RuHal₂〈 Diamin (I),

wobei Hal Chlor oder Brom bedeutet. Der Ru-Komplex kann durch Verknüpfung eines SH-Gruppen enthaltenden, anorganischen Trägers mit einem polymerisationsfähigen Bisphosphin(derivat) erhalten werden.

Eine besonders bevorzugte Verknüpfungsmethode besteht darin, eine radikalische Polymerisation eines Bisphosphin(oxid)s, das eine polymerisationsfähige Gruppe besitzt, in Gegenwart eines Kieselgels auszuführen, das SH-Gruppen enthält.

Derartige SH-gruppenhaltige Kieselgele sind bekannt und werden durch Modifizierung von Basiskieselgelen z.B. durch Umsetzung mit 3-Mercaptopropyl-trimethoxysilan unter saurer Katalyse erhalten.

Bei dieser Vorgehensweise ist die Belegungsdichte der Partikeloberfläche mit Katalysatorgruppierungen über den leicht einstellbaren Gehalt an SH-Gruppen auf dem Trägermaterial gut zu steuern. Zugleich ist es mit dieser Methode möglich, auch mit einer Polymerisationsreaktion eine hohe Bindungsausbeute, bezogen auf eingesetztes monomeres Bisphosphin(oxid), und eine hohe Belegungsdichte an fixierten Liganden zu erhalten. Heterogene Komplexkatalysatoren, die auf diese Weise hergestellt werden, zeichnen sich außerdem durch eine hohe Druckstabilität aus, die vor allem für den Einsatz in kontinuierlichen Verfahren eine wichtige Eigenschaft ist. Für diese Ausführungsart der Herstellung der neuen Katalysatoren für das erfindungsgemäße Verfahren eignen sich Bisphosphin(oxid)e mit polymerisationsfähigen Gruppen, insbesondere die von der Erfindung ebenfalls umfassten Monomere M¹, die nachfolgend formelmäßig beschrieben werden:

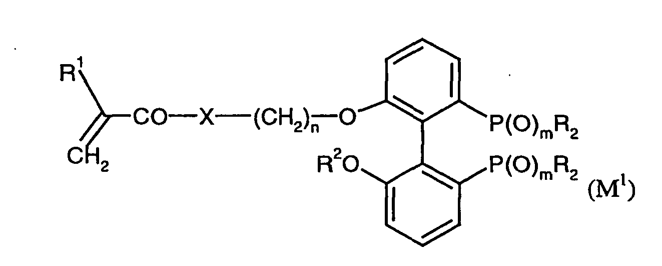

In der Formel M¹
steht
- R: für Phenyl, 2- oder 3- oder 4-Methylphenyl, 3,5-Dimethylphenyl, 3,5-Dimethyl-4-methoxyphenyl, 3,5-Di-tertbutylphenyl oder Cyclohexyl,
- R¹: steht für Wasserstoff oder Methyl,
- X: für O oder NH,
- R²: steht für Methyl, Ethyl, n- oder i-Propyl, n- oder i-Butyl, n-Pentyl, n-Hexyl, Cyclohexyl, n-Heptyl oder n-Octyl,
- n: steht für eine Zahl von 2 bis 12,
- m: steht für Null oder 1, vorzugsweise 1.

Die Herstellung eines Bisphosphinoxids, das durch die Formel M¹ beschrieben wird (wobei m = 1 ist), erfolgt beispielsweise gemäß Schema 1: 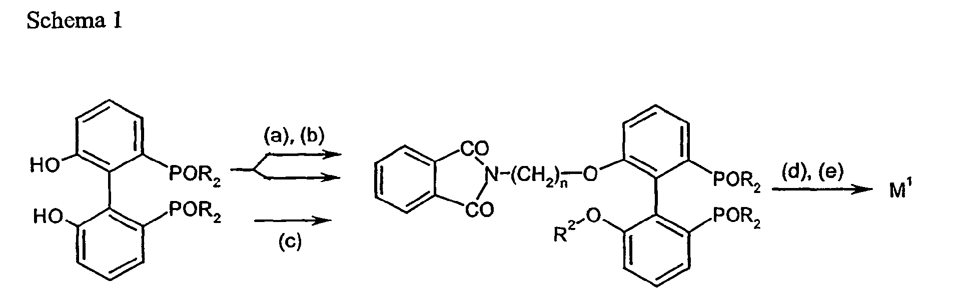
(a): R¹-Hal (Hal=Br, I)/K₂CO₃/DMF, 80° oder N-ω-Br-alkyl-phthalimid/ K₂CO₃/DMF, 80°
(b): N- ω-Bromalkyl-phthalimid/K₂CO₃/DMF, 80° oder R¹-Hal(Hal = Br, I)
(c): N- ω-Bromalkyl-phthalimid/ R¹-Hal(Hal = Br, I)/K₂CO₃/DMF, 80°
(d): N₂H₄, EtOH, reflux/HCL, H₂O
(e): CH₂ = CR1COCI/NaOH/CH₂Cl₂H₂O.

Beide Synthesealternativen sind verwendbar, bevorzugt ist die einstufige, gemischte Alkylierung (c).

Eine weitere bevorzugte Gruppe von Monomeren leitet sich von neuen Bisphosphin(oxiden) der Formel M² ab, deren Herstellung und weitere Umsetzungen in Schema 2 dargestellt ist. 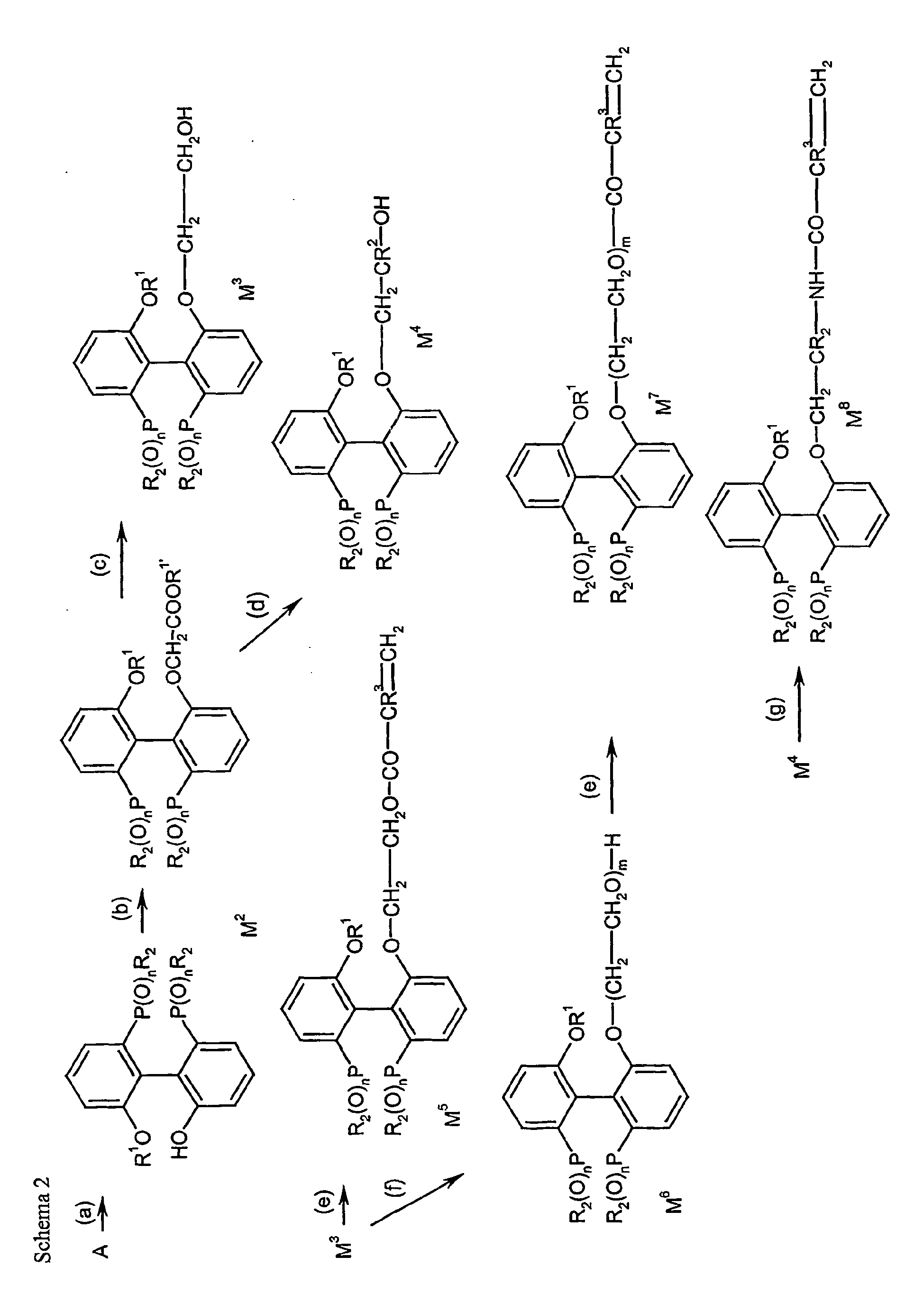 A steht in Schema 2 für (R)- oder (S)-(6,6'-Dihydroxy-biphenyl-2,2'-diyl)bis(diphenylphosphin) oder deren Bisphosphinoxide, vorzugsweise für die Bisphosphinoxide.
In den Formeln des Schema 2 stehen
- R: für Phenyl, 2- oder 3- oder 4-Methylphenyl, 3,5-Dimethylphenyl, 3,5-Dimethyl-4-methoxyphenyl, 3,5-Ditert. butylphenyl oder Cyclohexyl,
- R^{1'}, R¹ und R²: unabhängig voneinander für C₁- bis C₈-(Cyclo)Alkyl with Methyl, Ethyl, n- oder i-Propyl, n-, i- oder sec. Butyl, 2,2-Dimethyl-1-butyl, Cyclohexyl, n-Heptyl und n-Octyl.
- R³: steht für H oder CH₃, n für 1 oder Null, m für 2-100, vorzugsweise 2-60.

Legende zu Schema 2:
(a): R¹ Br/K₂CO₃/DMF, 80°;
(b): BrCH₂COOR¹/K₂CO₃/DMF, 80°;
(c): LiAlH₄, THF/ggf. nachfolgend H₂O₂, CH₂Cl₂;
(d): R²MgX (X=Br oder J), THF;
(e): CH₂=CR³COCl, Base
(f): Ethylenoxid, R¹ONa(kat.);
(g): CH₂=CR³CN, H₂SO₄.

Die Verbindungen der allgemeinen Formeln M¹, M², M³, M⁴, M⁵ und M⁶ sind von der Erfindung ebenfalls umfasst.

Die polymerisationsfähigen Monomeren M⁵ und M⁶ sind jeweils Mischungen von Diastereomeren, die durch die beschriebene Verknüpfung mit entsprechend funktionalisierten Trägern zu wertvollen Katalysatoren fähren, die in das erfindungsgemäße Verfahren eingesetzt werden.

Gewünschtenfalls können diese Mischungen nach bekannten Verfahren, z.B. durch fraktionierte Kristallisation oder chromatographisch, in die einzelnen Stereoisomeren aufgetrennt und in die entsprechenden Katalysatoren umgewandelt werden.

Das überbrückte Bisphosphinoxid der Formel M² ist ein wertvolles Zwischenprodukt, das durch Epoxidation oder Dihydroxylierung in entsprechend funktionalisierte Derivate umgewandelt werden kann, die nach Verknüpfung mit geeigneten Trägern, z.B. Amino- oder Carboxyl-Gruppen enthaltenden Reaktivharzen, zu erfindungsgemäßen Katalysatoren führen.

In gleicher Weise können die Aminogruppen enthaltenden Bisphosphinoxide M9 und M¹⁰ verwendet werden. Es kann vorteilhaft sein, die entsprechenden Bisphosphine M^{9'} und M10^{'}, die ebenfalls von der Erfindung umfasst sind, für die Verknüpfung mit entsprechend funktionalisierten Trägern einzusetzen, weil die in bekannter Weise durch Reduktion mit Trichlorsilan zugänglichen monomeren Bisphosphine vielseitiger verwendet werden können als ein in bestimmter Weise fixiertes Phosphin.

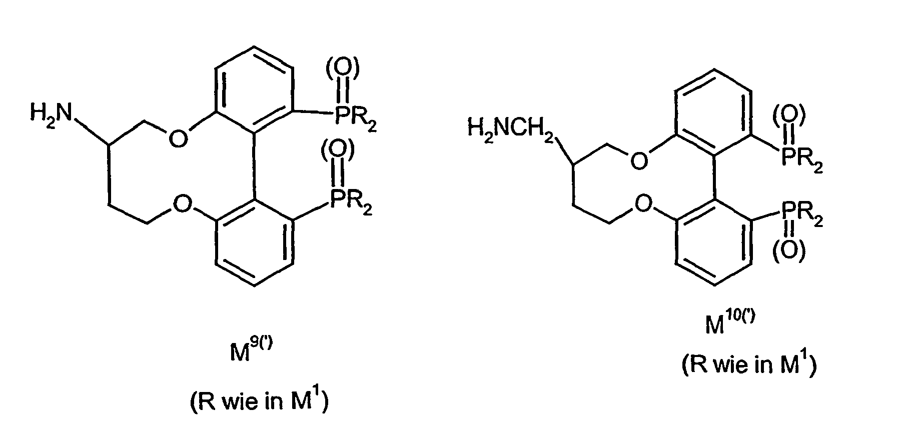

Es hat sich gezeigt, dass nicht nur Katalysatoren der Formel (I), die die Bausteine M^{9'} oder M^{10'} enthalten, hervorragende Katalysatoren für die enantioselektive Hydrierung von (einfachen) Ketonen sind, sondern vielmehr auch, dass ihre Vorstufen der Formel (II) überraschenderweise als hervorragend selektiv und aktiv wirkende Hydrieroder Isomerisierungs-Katalysatoren für andere Substrate, wie z.B. β-Ketacarbonsäureester, α,β-ungesättigte Carbonsäuren oder bestimmte Allylamine in an sich bekannter Verfahrensweise eingesetzt werden können.

Es liegen dann an einen Träger gebundene Bisphosphine vor.

Es sei darauf hingewiesen, dass beliebige Kombinationen von Vorzugsbereichen ebenfalls von der Erfindung umfasst sind.

Um zu erfindungsgemäß zu verwendenden heterogenen Ru(II)-Phosphin-KomplexKatalysatoren der Formel (II) zu gelangen, kann man die an einen Träger gebundenen Phosphine mit geeigneten Ru(II)-Komplexen umsetzen. Als Ru(II)- Komplexe kommen beispielsweise dafür die Komplexe der Formel

[Ru(aren)X₂]₂,

in der
X für Cl oder Br
steht,
zum Einsatz, wie z.B. (p-Cymol)-ruthenium(II)chlorid, dimer, (siehe J. Org. Chem., 59, 3064, 1994). Insbesondere ist auch der Bis-(2-methallyl-cycloocta-1,5-dien-Ru(II)-Komplex für die Herstellung von Katalysatoren der Formel (II) geeignet, (siehe Tetrahedron: Asymmetry, Vol. 2, No. 7, S. 565, 1991).

Zur Herstellung von Katalysatoren der Formel (I) wird der heterogene Precursor der Formel (II) in Lösungen des Diamins suspendiert. Als Lösungsmittel werden dafür beispielsweise Dichlormethan, Acetonitril oder DMF verwendet. Man kann 1 bis 10 Äquivalente des Diamins bezogen auf Ru in verdünnter Lösung einsetzen und die Umsetzung unter Schutzgas, vorzugsweise Argon, bei Temperaturen von 20°C bis 100°C im Verlauf von etwa 3 bis 48 Stunden ausführen. Der unter Schutzgas abfiltrierte und ausgewaschene Katalysator der Formel (I) kann im Vakuum getrocknet werden und ist lagerstabil.

Als Carbonylverbindungen zum Einsatz für das erfindungsgemäße Verfahren kommen z.B. solche der Formel (V) in Frage

R¹-CO-R² (V),

in der
R¹ und R² gleich oder verschieden sein können und jeweils für Wasserstoff, für geradkettiges oder verzweigtes C₁-C₁₂-Alkyl, C₂-C₁₂-Alkenyl oder C₂-C₁₂-Alkinyl, für C₂-C₈-Cycloalkyl, für C₆-C₁₂-Aryl oder für C₄-C₁₁-Heteroaryl mit jeweils 1 bis 3 Ring-Heteroatomen aus der Gruppe N, O oder S stehen.

Alkyl-, Alkenyl-, Alkinyl- und Cycloalkylreste können gegebenenfalls mit Halogen, Hydroxy, Di-C₁-C₁₂-alkylamino-, C₆-C₁₀-Aryl-C₁-C₁₂-alkylamino-, Di-C₆-C₁₀-arylamino-, C₁-C₁₂-Alkoxy-, C₁-C₁₂-Alkoxycarbonyl-, Amid- und/oder UrethanGruppen substituiert sein, wobei beispielsweise bis zu 3 gleiche oder verschiedene Substituenten vorliegen können.

Aryl- und Heteroarylreste können gegebenenfalls mit C₁-C₁₂-Alkyl-, Di-C₁-C₁₂-alkylamino-C₁-C₁₂-alkyl-, Halogen-C₁-C₁₂-alkyl-, Hydroxy-C₁-C₁₂-alkyl-, C₂-C₁₂-Alkenyl-, C₂-C₁₂-Alkinyl-, Halogen-, C₁-C₁₂-Alkoxy-, Halogen-C₁-C₁₂-Alkoxy-, C₆-C₁₀-Arakoxy-, Hydroxy-, Carboxyl-, C₁-C₁₂-Alkoxycarbonyl-, Amid- und/oder Urethangruppen substituiert sein, wobei beispielsweise bis zu 3 gleiche oder verschiedene Substituenten vorliegen können.

R¹ und R² können gemeinsam mit der dazwischenliegenden CO-Gruppe auch ein Cyclo-C₄-C₁₂-alkylketon bilden, wobei der Cycloalkylteil gegebenenfalls wie oben für R¹ = Alkyl angegeben, substituiert und gegebenenfalls auch ungesättigt sein kann.

Bei den Alkylgruppen, auch in kombinierten Resten, handelt es sich vorzugsweise um C₁-C₆-Alkylgruppen. Bei den Alkenyl- und Alkinylgruppen, auch in kombinierten Resten, handelt es sich vorzugsweise um C₂-C₄-Alkenyl- bzw. C₂-C₄-Alkinylgruppen.

Bei den Cycloalkylgruppen, auch in kombinierten Resten, handelt es sich vorzugsweise um C₄-C₇-Cycloalkylgruppen.

Bei den Arylgruppen, auch in kombinierten Resten, handelt es sich vorzugsweise um C₆-C₁₀-Arylgruppen, bei den Heteroarylgruppen vorzugsweise um solche, die 5 bis 9 Ring-C-Atome enthalten.

Bei den Alkoxygruppen in kombinierten Resten handelt es sich vorzugsweise um C₁-C₆-Alkoxygruppen.

Bei Halogen in kombinierten Resten handelt es sich vorzugsweise um Fluor oder Chlor.

Besonders bevorzugte Alkylgruppen sind:

Methyl, Ethyl, Propyl, Isopropyl, n-Butyl, Isobutyl, tert.-Butyl, sec.-Butyl, Pentyl, Hexyl, Heptyl, Chlormethyl, 2-Chlorethyl, 2-Hydroxyethyl, 2-Dibenzylaminoethyl-, 2-(N-Benzyl-N-methylamino)-ethyl, 2-Ethoxyethyl, Methoxycarbonylmethyl, 2-(N-Methyl-N-methoxycarbonylamino)-ethyl, Vinyl, Methallyl, Propinyl, Cyclopropyl, Cyclobutyl, Cyclopentyl, Cyclohexyl, 2-Methyl-cyclohexyl, Benzyl, Pyridyl-2-methyl und (5-Trifluormethyl-pyridyl-2)-methyl.

Besonders bevorzugte Arylgruppen sind:

Phenyl, 2-Methylphenyl, 2-Ethylphenyl, 2-Isopropylphenyl, 2-tert.-Butylphenyl, 3-Pentylphenyl, 4-Isobutylphenyl, 2,3-Dimethylphenyl, 2,4,6-Trimethylphenyl, 2-(2-Dimethylaminoethyl)-phenyl, 2-Trifluormethylphenyl, 4-(2-Hydroxyethyl)-phenyl, 3-Vinylphenyl, 4-(Propinyl-1)-phenyl, 4-Benzylphenyl, 2-Chlorphenyl, 3-Fluorphenyl, 2-Methoxyphenyl, 3,4-Dimethoxyphenyl, 4-Benzyloxyphenyl, 1-Naphthyl, 2-Naphthyl und 2-Indenyl.

Besonders bevorzugte Hetarylgruppen sind:

Pyridyl, Pyrimidyl, Pyrazolyl, Imidazolyl, Thienyl, Furyl, Oxazolyl und Indolyl, wobei als Substituenten solche in Frage kommen, die oben bei besonders bevorzugten Arylgruppen genannt worden sind.

Besonders bevorzugte Cyclo-C₄-C₁₂-alkylketone sind:

Cyclobutanon, Cyclopentanon, Cyclohexanon, 4-Methyl-cyclohexanon, 2-Methyl-cyclohexanon, 2-tert.-Butyl-cyclohexanon, 4-tert.-Butyl-cyclohexanon, Cyclohexanon und 2,4,4-Trimethyl-2-cyclohexanon.

Als Basen können in das erfindungsgemäße Verfahren beispielsweise Hydroxide oder Alkoholate von Alkalimetallen oder quaternäre Ammoniumhydroxide eingesetzt werden. Es handelt sich dabei insbesondere um Lithium-, Natrium- oder Kaliumhydroxide, Lithium-, Natrium- oder Kalium-C₁-C₄-alkylalkoholate oder Tetra-C₁-C₄-alkylammoniumhydroxide. Besonders bevorzugt sind Kaliumhydroxid, Lithiumhydroxid, Kaliummethylat, Natriummethylat, Natriumisopropylat, Kalium-tert.-butylat, Tetramethylammoniumhydroxid und Tetrabutylamoniumhydroxid.

Für die Herstellung des Katalysators der Formel (I) sind Diamine geeignet, die mit Ru(II) einen Chelatkomplex bilden können. Beispielsweise seien genannt: 1,2-Diaminoethan, 1,2- und 1,3-Diaminopropan, 1,2-Diaminobutan, 2,3-Diaminobutan, 2,3-Diaminopentan, 1,2-Diamino-1,2-diphenylethan, 1,2-Diaminocyclopentan, 1,2-Diaminocyclohexan, 1,2-Diamino-methyl-cyclohexan, 1-Amino-2-N-methylamino-ethan und 1-Amino-1-methyl-2-N-methylaminocyclohexan.

Bevorzugte, optisch aktive Amine zur Herstellung der trägergebundenen Katalysatoren der Formel (I) sind chiral einheitliche Diamine, insbesondere solche, die sich vom 1,2-Diamino-ethan und vom 1,2-Diaminocyclohexan ableiten und gegebenenfalls C₁-C₈-Alkyl, C₄-C₈-Cycloalkyl-, C₆-C₁₀-Aryl-C₁-C₈-alkyl, C₂-C₈-Alkenyl- und/oder gegebenenfalls durch C₁-C₈-Alkyl und/oder C₁-C₈-Alkoxy substituierte C₆-C₁₀-Arylgruppen als Substituenten enthalten können.

Besonders bevorzugt werden für die Herstellung der neuen Katalysatoren der Formel (I) die Diamine der Formeln (ITI) und (IVa-c):

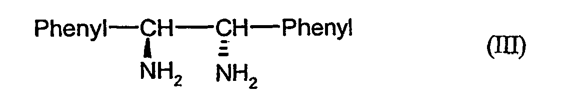

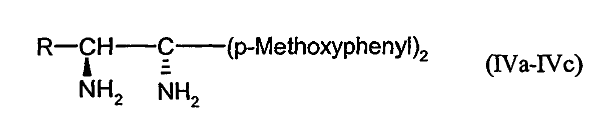

(IVa): R = CH₃

(IVb): R = CH(CH₃)₂

(IVc): R = CH₂-CH(CH₃)₂

Für die erfindungsgemäße Herstellung von optisch aktiven Alkoholen können diese optisch aktiven Amine sowohl als (S,S)-, (R,R)-, (R)- oder (S)-Stereoisomer eingesetzt werden.

Diese Stereoisomeren können auf bekannte Weise oder analog dazu hergestellt werden (siehe z.B. Tetrahedron, Lett. 34 (12), 1905 (1993). Welches optisch aktive Amin in welcher Form in Kombination mit einem bestimmten erfindungsgemäß einzusetzenden Katalysator bei der erfindungsgemäßen Herstellung eines bestimmten optisch aktiven Alkohols optimale Ergebnisse erbringt, kann gewünschtenfalls durch routinemäßige Reihenversuche gemäß der "in situ"-Variante des Verfahrens ermittelt werden.

Bei der Ausführung des erfindungsgemäßen Verfahrens gemäß der "in situ-Variante" liegt bei diskontinuierlicher Arbeitsweise, z.B. in einem Rührautoklaven, die Menge eines Katalysators der Formel (II), berechnet als Mole Ru(II), pro Mol Carbonylverbindung im Bereich von 1:100 bis 1:100 000. vorzugsweise liegt diese Menge bei 1:200 bis 1:10 000.

Das Diamin kann, bezogen auf heterogenen Ru(II)-Phosphin-Komplex-Katalysator(en), (berechnet als Mole Ru(II)) beispielsweise in Mengen von 1:0,5 bis 1:4 eingesetzt werden. Vorzugsweise liegt diese Menge bei 1:1 bis 1:2,5 pro Mol Ru(II). Die Base kann, bezogen auf den heterogenen Ru(II)-Phosphin-Komplex-Katalysator (berechnet als Mole Ru(II)) beispielsweise in Mengen von 0,5 bis 1000 Äquivalenten eingesetzt werden. Vorzugsweise liegt diese Menge bei 2 bis 40 Äquivalenten Base pro Mol Ru(II).

Wird das erfindungsgemäße Verfahren unter Verwendung eines gesondert isolierten bereiteten Katalysators der Formel (I) ausgeführt, kann die Menge des Katalysators (berechnet als Äquivalente Ru(II) pro Mol eingesetzte Carbonylverbindung), 1:100 bis 1:500 000 betragen. Vorzugsweise liegt diese Menge bei 1:1 000 bis 1:200 000.

Im Falle der Verwendung von Katalysatoren der Formel (I) ist ein Zusatz von Diamin zum Reaktionsgemisch bzw. zur Lösung des Substrates nicht notwendig, kann aber zur Steigerung der Lebensdauer des heterogenen Katalysators vorteilhaft sein.

Die Menge eines solchen Zusatzes von Diamin liegt im Bereich von 0,01 bis 1,0 Äquivalenten, bezogen auf eingesetzte Mole Ru(II)-Komplex.

Für die eingesetzten Mengen an Base gelten die gleichen Verhältnisse, die oben für die in-situ-Variante angegeben sind.

Es ist vorteilhaft, das erfindungsgemäße Verfahren in Gegenwart von Lösungsmitteln durchzuführen. Als Lösungsmittel sind solche geeignet, die mit den eingesetzten Materialien nicht in unerwünschter Weise reagieren und ein ausreichendes Lösevermögen für die eingesetzte Carbonylverbindung und das eingesetzte Amin haben. Beispiele sind aliphatische Kohlenwasserstoffe wie Hexan und Isooctan, aromatische Kohlenwasserstoffe wie Toluol und die Xylole, halogenhaltige Kohlenwasserstoffe wie Methylenchlorid, lineare und cyclische aliphatische Ether wie tert.-Butyl-methylether und Tetrahydrofuran, C₁-C₈-Alkyl und C₇-C₁₀-Aralkyl-alkohole wie Methanol, Ethanol, n-Propanol, i-Propanol und Benzylalkohol und dipolar-aprotische Lösungsmittel wie Acetonitril, Dimethylformamid und N-Methylpyrrolidon.

Bevorzugte Lösungsmittel sind C₁-C₄-Alkylalkohole, insbesondere i-Propanol. Es können auch Lösungsmittel-Gemische eingesetzt werden.

Man kann ohne Lösungsmittelzusatz arbeiten oder mit Lösungsmittelzusätzen bis hinunter zu einer Substratkonzentration von 1 Gew.-% oder weniger. Vorzugsweise nimmt man soviel Lösungsmittel, dass sich eine Substratkonzentration im Bereich von 10 bis 50 Gew.-% ergibt.

Der beim erfindungsgemäßen Verfahren anzuwendende Wasserstoffdruck kann z.B. zwischen 1 und 150 bar betragen. Vorzugsweise liegt er im Bereich von 3 bis 120 bar, insbesondere zwischen 5 und 100 bar.

Die Reaktionstemperatur kann beim erfindungsgemäßen Verfahren z.B. im Bereich von -20 bis +120°C liegen. Vorzugsweise liegt sie in einem Bereich von +15 bis +100°C, insbesondere von +25 bis +100°C.

Die Reaktionszeit ist abhängig von der Ausführungsform des Verfahrens und den Reaktionsbedingungen. Sie liegt im allgemeinen in einem Bereich von beispielsweise 5 Minuten bis 12 Stunden.

Beim erfindungsgemäßen Verfahren ist die Aufarbeitung des Reaktionsgemisches einfach, da man den Katalysator beispielsweise durch Filtration und die im Reaktionsgemisch vorhandenen Basen und Amine mit Hilfe eines Ionenaustauschers entfernen kann. Der isolierte Katalysator kann wiederverwendet werden. Die hergestellten, gegebenenfalls optisch aktiven Alkohole, sind nach der Aufarbeitung des Reaktionsgemisches nicht mit Katalysatoren oder deren Bestandteilen verunreinigt. Das erfindungsgemäße Verfahren kann auch problemlos kontinuierlich durchgeführt werden.

Überraschenderweise zeigt das erfindungsgemäße Verfahren Selektivitäten und Aktivitäten der verwendeten Katalysatoren, die im Bereich der Werte homogener Katalysatoren liegen.

### Beispiele

### Beispiel 1

In einem 250 ml Rührautoklaven wurde eine Lösung von 12 g Acetophenon in 100 ml i-Propanol unter Zusatz von 500 mg eines trägergebundenen Rutheniumkomplexes der Formel (IIa) 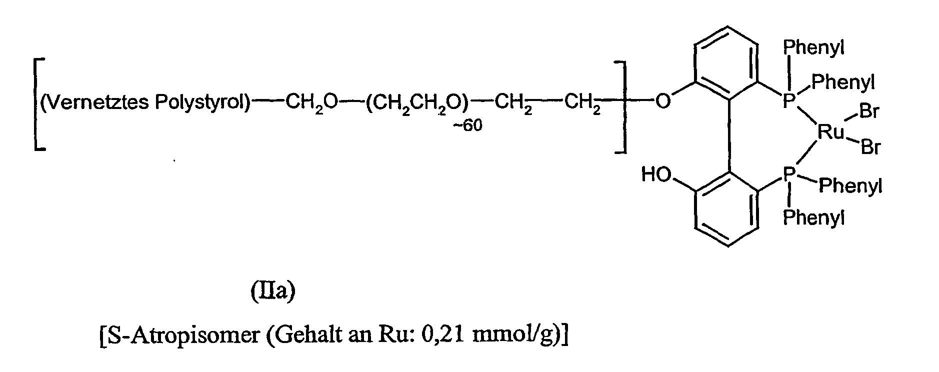 39 mg (S)-1,1-Di-(p-Anisyl)-3-Methyl-1,2-Diamino-butan und 420 µl einer 0,5-molaren Lösung von Kaliumhydroxid in i-Propanol mehrfach unter Gefriertrocknungsbedingungen entgast ("freeze-thaw-cycles") und die Gasphase durch Wasserstoff ersetzt. Anschließend wurde bei 40°C 6 Stunden lang 50 bar Wasserstoff aufgedrückt. Danach wurde unter Schutzgas filtriert, die verbleibende Reaktionslösung mit einem sauren Ionenaustauscherharz behandelt, wobei das Diamin und Kaliumionen gebunden wurden. Nach der Filtration wurde das beladene Austauscherharz mehrfach mit i-Propanol gewaschen und die Produktlösung zusammen mit den Waschphasen wurden destilliert. Es wurden 11,6 g über 99 % reines 1-Phenyl-Ethanol mit einem Gehalt von 90 % an R-Enantiomer erhalten (CSP-HPLC-Analyse).

Der wiedergewonnene, trägergebundene Rutheniumkomplex und das mittels Ionenaustausch abgetrennte und wiedergewonnene Diamin wurden in einem weiteren Herstellungsverfahren entsprechend Beispiel 1 anstelle von frischem Katalysator und frischem Diamin eingesetzt. Es wurden nahezu identische Ergebnisse erhalten.

### Beispiel 2

Herstellung des in Beispiel 1 verwendeten Ru-Komplexes
a) 0,5 g (S)-6,6'-Dihydroxydiphenyl-2,2'-diyl-bis-(diphenylphosphin), hergestellt gemäß WO 93/15090, Beispiel 1, wurden unter Argon in 50 ml wasserfreiem und entgastem Tetrahydrofuran gelöst und eine Suspension von 0,216 g Natriumhydrid in 10 ml Dimethylformamid gegeben. Das Gemisch wurde 60 Minuten bei Raumtemperatur gerührt. Dann wurden 4 g TentaGel^{®} S-Bromid¹ hinzugefügt und die Mischung weitere 48 Stunden bei Raumtemperatur gerührt. Danach wurde der vorliegende Feststoff abfiltriert, mit gesättigter wässriger Amoniumchlorid-Lösung und danach dreimal mit je 50 ml wasserfreiem Methanol ausgerührt und abfiltriert. Nach der letzten Filtration wurde das erhaltene Produkt im Vakuum getrocknet.
   ¹ TentaGel-Reaktivharze (Produkte der Rapp Polymere GmbH, Tübingen, Deutschland), sind Copolymere, die durch stufenweise Propfung einer vernetzten Polystyrolmatrix mit Polyethylenglykol und Ethylenoxid gemäß EP 187 391 erhalten werden. sie enthalten frei bewegliche Endgruppen, z.B. im Fall von TentaGel S-Br die Gruppierung CH₂-CH₂-Br.
b) 800 mg des nach a) erhaltenen modifizierten Trägerharzes und 53 mg Bis-(2-methallyl)-cycloocta-1,5-dien-Ru(II)-Komplex wurden unter Argon in 20 ml wasserfreiem und entgastem Aceton suspendiert und unter Rühren gelöst. Dann wurden 1,38 ml 0,29 molare Bromwasserstofflösung hinzugeführt. Die Mischung wurde 2 Stunden bei Raumtemperatur gerührt, anschließend unter Argon filtriert. Der erhaltene Feststoff wurde unter Argon mit Aceton, abschließend mit i-Propanol gewaschen, bis das Filtrat frei von Ruthenium war. Nach dem Trocknen im Vakuum ergab eine Rutheniumanalyse eine Beladung von 0,21 mmol/g.

### Beispiel 3

In einem 250 ml Rührautoklaven wurde eine Lösung von 12 g Acetophenon in 100 ml i-Propanol unter Zusatz von 40 mg eines trägergebundenen Rutheniumkomplexes der Formel (Ia) 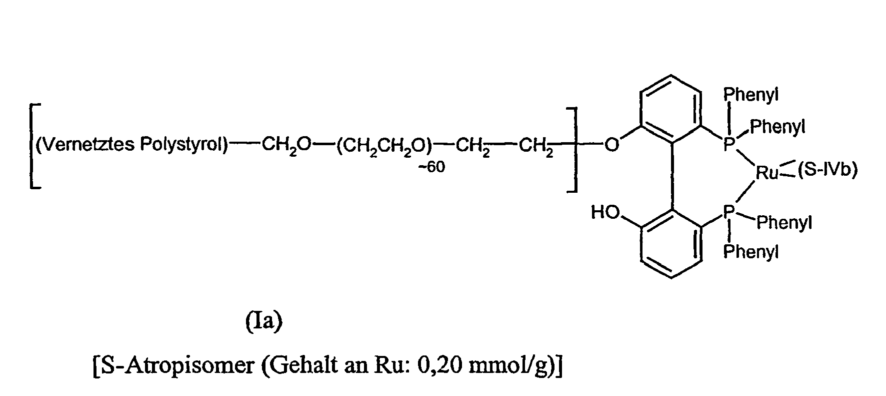 und von 420 µl einer 0,5 molaren Lösung von Kaliumhydrid in i-Propanol mehrfach unter Gefriertrocknungsbedingungen ("freeze-thaw-cycles") entgast und die Gasphase durch Wasserstoff ersetzt. Unter Rühren wurde anschließend bei 40°C bei einem Wasserstoffdruck von 40 bar 2 Stunden lang hydriert. Nach Filtration und Auswaschen des als Filterrückstand verbliebenen Katalysators mit 10 ml i-Propanol wurde das Filtrat, vereinigt mit der Waschlösung, im Vakuum destilliert, wobei 11,7 g reines 1-Phenyl-ethanol mit einem Gehalt von 90 % an R-Enantiomer erhalten wurden (CSP-HPLC-Analyse).

### Beispiel 4

Herstellung des in Beispiel 3 verwendeten Katalysators der Formel (Ia)
1 g des gemäß Beispiel 2 hergestellten Katalysators der Formel (IIa) wurde unter Argon zu einer entgasten Lösung von 120 mg (S)-1, 1-Di-(p-anisyl)-3-methyl-1,2-diamino-butan in 20 ml Dichlormethan gegeben und die Mischung unter Rühren 12 Stunden bei 25°C gehalten. Nach Filtration unter Schutzgas wurde der erhaltene Katalysator der Formel (Ia) mit 20 ml Dichlormethan ausgewaschen und anschließend im Vakuum getrocknet.

### Beispiel 5

In drei Rührautoklaven, die zu einer Rührkesselkaskade mit einem effektiven Gesamtvolumen von 1,51 verbunden wurden, die jeweils einen durch Sintermetallfritten mit einer Porengröße von 10 µm abgesperrten Überlauf enthielten, wurde eine Gesamtmenge von 30 g des nach Beispiel 4, erhaltenen heterogenen Bisphosphin-Diamin-Ru-Komplex-Katalysators - in jeweils gleicher Menge unter Argon in drei abgeschmolzenen Glasampullen abgefüllt - in die Autoklaven eingebracht.

Nach Befüllung der zuvor mit Argon gespülten Apparatur mit reinem 2-Propanol und Zuführung von Wasserstoff unter einem Druck von 10 bar wurden die Rührer in Gang gesetzt (wobei die Glasampullen aufgebrochen wurden und der Katalysator suspendiert wurde) und zugleich wurde pro Stunde eine Lösung von 20 g 1-Acetylnaphthalin (1'-Acetonanphone) in 250 ml 2-Propanol, der außerdem 2,3 ml einer 1,0 M Kaliumtert.butylat-Lösung in tert. Butanol zugesetzt waren, kontinuierlich zugepumpt.
Die Reaktionstemperatur wurde bei 25°C gehalten.

Analytische Untersuchungen (GC und CSP-HPLC) von Proben, die nach bestimmten Betriebszeiten der Hydrierapparatur erhalten wurden, ergaben nach 10 Stunden kontinuierlichem Betrieb einen Umsatz von 98 % und eine Enantioselektivität von 97 % ee, nach 100 Stunden einen Umsatz von 100 % und eine Enantioselektivität von 98 % ee, nach 240 Stunden kontinuierlicher Betriebszeit einen Umsatz von 99 % und eine Enantioselektivität von 98 % ee für das Hydrierprodukt [1-(α-Naphthyl)-ethanol].

### Beispiel 6

4,0 g (S)-(6,6'-Dihydroxybiphenyl-2,2'-diyl)bis(diphenylphosphinoxid), 3,65 g Kaliumcarbonat und 5,8 g Cyclohexylbromid wurden zu 70 ml N,N-Dimethylformamid gegeben und die Mischung über einen Zeitraum von 60 Stunden bei 80°C gerührt. Nach Abkühlen wurde unverändertes (S-(6,6'-Dihydroxybiphenyl-2,2'-diyl)bis(diphenylphosphinoxid) (3,2 g) und anorganische Salze zentriert. Zu der klaren Lösung des Produktes wurden 100 ml Wasser gegeben und die Mischung dann 3 x mit 30 ml Chloroform extrahiert. Die abgetrennte organische Phase wurde getrocknet, mit Hilfe eines Rotationsverdampfers eingeengt und das Rohprodukt durch Chromatographie gereinigt (Silikagel Merck 60, Laufmittel: Ethylacetat/- Methanol/Wasser 75:3:1,5). Es wurden 700 mg reines (S)-(6-Cyclohexyl-oxy-6'-hydroxybiphenyl-2,2'-diyl)bis(diphenylphosphinoxid) erhalten.
Schmelzpunkt: 159 bis 162°C [α_{D}] = -98,7°C (c = 1,7, Chloroform).

### Beispiel 7

280 mg (S)-(6-Cyclohexyloxy-6'-hydroxybiphenyl-2,2'-diyl)bis(diphenylphosphinoxid), 260 mg Kaliumcarbonat und 0,3 ml Bromessigsäuremethylester wurden zu 10 ml N,N-Dimethylformamid gegeben. Die Mischung wurde über einen Zeitraum von 12 Stunden bei 80°C gerührt. Nach Abkühlen wurden der Mischung 30 ml Wasser zugegeben. Danach wurde die Mischung 3 x mit 30 ml Chloroform extrahiert. Die abgetrennten organischen Phasen wurden vereinigt über Magnesiumsulfat getrocknet und mit Hilfe eines Rotationsverdampfers eingeengt. Nach flash-Chromatographie an Silikagel wurden 291 mg (S)-(6-Cyclohexyloxy-6'-methoxycarbonylmethoxybiphenyl-2,2'-diyl)bis(diphenylphosphinoxid) erhalten.
[α_{D}] = -68,1°C (c = 1,1, Chloroform).

### Beispiel 8

4 g (S)-(6-Cyclohexyloxy-6'-methoxycarbonylmethoxybiphenyl-2,2'diyl)bis-(diphenylphosphinoxid) (analog zu Beispiel 7 hergestellt) wurden in 50 ml trockenem THF gelöst. Das Methyl Grignard Reagenz, das aus 310 mg Magnesiumspänen und 1,76 g Methyliodid in 80 ml THF erhalten wurde, wurde über einen Tropftrichter innerhalb von 30 Minuten zugegeben. Dann wurde die Mischung unter Rühren für 8 Stunden auf 50°C erhitzt. Nach Abkühlen wurden 250 ml Wasser zugesetzt und die Mischung mit 2 N Salzsäure angesäuert und 3 x mit Chloroform (50 ml) extrahiert. Die abgetrennten organischen Phasen wurden vereinigt, über Natriumsulfat getrocknet und mittels Rotationsverdampfer eingeengt. Das Produkt wurde durch Chromatographie weiter gereinigt (Silikagel Merck 60, Laufmittel: Ethylacetat/Methanol/Wasser 75:3:1,5). Ausbeute: 3,7 g (S)-[6-Cyclohexyloxy-6'-(2-hydroxy-2-methylpropyloxy)biphenyl-2,2'-diyl]bis(diphenylphosphindioxid).

### Beispiel 9

3,7 g (S)-[6-Cyclohexyloxy-6'-(2-hydroxy-2-methylpropyloxy)biphenyl-2,2'-diyl]-bis(diphenylphosphindioxid) wurden zu 75 ml Acrylnitril gegeben. Über einen Tropftrichter wurden innerhalb von 30 Minuten bei 0 bis 5°C 10 ml konzentrierte Schwefelsäure unter Rühren zu der Lösung gegeben, dann wurde die Mischung für 5 Stunden bei 25°C gerührt. Nach Abkühlen auf 0°C wurden 300 ml Wasser zugegeben und die Mischung 3 x mit jeweils 100 ml Chloroform extrahiert. Die abgetrennten organischen Phasen wurden vereinigt, über Natriumsulfat getrocknet und am Rotationsverdampfer eingeengt. Das Produkt wurde durch Chromatographie gereinigt (Silikagel Merck 60, Laufmittel: Ethylacetat/Methanol/Wasser 75:3:1,5). Ausbeute: 3,3 g von (S)-[6-(2-acrylamido-2-methylpropyloxy)-6'cyclohexyloxybiphenyl-2,2'-diyl]bis(diphenylphosphinoxid).

### Beispiel 10

a) 30 g eines YMC-Silikagels mit einer durchschnittlichen Partikelgröße von 20 µmm 3 g 3-mercapto-trimethoxysilan, 0,9 g p-Toluolsulfonsäure-Monohydrat und 0,2 ml Wasser wurden zu 300 ml Toluol gegeben und die Mischung über einen Zeitraum von 8 Stunden unter Rühren zum Rückfluss erhitzt. Nach Abkühlen wurde das modifizierte Silikagel filtriert und mit Methylenchlorid/Methanol (1:1) und 2 x mit Methylenchlorid gewaschen und schließlich unter Hochvakuum bei 40°C getrocknet. Ausbeute: 31,8 g; Analyse S-Gehalt: 1,2 %.
b) 30 g des nach Beispiel 10 a) erhaltenen modifizierten Silikagels, 3 g (S)-[6-(10-N-methylacrylamido-decyloxy)-6'-cyclohexyloxybiphenyl-2,2'-diyl]bis-(diphenylphosphinoxid), 3 g frisch destilliertes Styrol und 60 mg AIBN wurden zu 50 ml Toluol gegeben. Die Mischung wurde über einen Zeitraum von 12 Stunden bei 60°C gerührt, anschließend wurden 0,4 g 2,2-Methylen-bis(6-tert.butyl-4-methylphenol) und 3 ml Bis-trimethylsilylacetamid zugegeben und die Mischung bei 100°C über einen Zeitraum von 4 Stunden gerührt. Nach Abkühlen wurde das gepfropfte Silikagel abfiltriert und nacheinander mit je 30 ml Methylenchlorid, Methylenchlorid/Methanol (1:1), Toluol, Isopropanol und wieder mit Methylenchlorid gewaschen. Das gepfropfte Silikagel wurde dann im Hochvakuum bei 40°C getrocknet. Ausbeute: 34,4 g; Analyse P-Gehalt: 0,31 % =̂ 0,050 mmol Diphosphinoxid/g Silikagel.
c) 30 g des nach Beispiel 10 b) erhaltenen modifizierten Silikagels, 15,5 ml Tributylamin und 3,85 ml Trichlorsilan wurden unter Argonatmosphäre zu 100 ml Xylol gegeben. Die resultierende Mischung wurde unter Rühren für 24 Stunden unter Rückfluss erhitzt. Nach Abkühlen auf Raumtemperatur wurde das Silikagel abgefiltert (alle Operationen wurden unter Argon durchgeführt). Abschließend wurde das Silikagel in 100 ml Dichlormethan subspendiert und der Mischung unter Rühren 30 ml einer 4 N wässrigen Natriumhydroxidlösung zugegeben. Diese Mischung wurde über einen Zeitraum von 1 Stunde bei Raumtemperatur gerührt. Dann wurde das Silikagel erneut abfiltriert und mit jeweils 100 ml Dichlormethan/Methanol (1:1) Toluol, Isopropanol und schließlich Dichlormethan gewaschen. Das modifizierte Silikagel wurde im Hochvakuum bei 40°C getrocknet. Ausbeute: 29,7 g; Analyse P-Gehalt: 0,32 %, =̂ 0,051 mmol Diphosphin/g Silikagel.

### Beispiel 11

29,7 g des nach Beispiel 10 c) erhaltenen Silikagels und 1,176 g [RuCl₂(η⁶-benzol] (= 2,35 mmol) wurden unter Argon zu 150 ml entgastem DMF gegeben. Dann wurde die Mischung unter Rühren für 24 Stunden auf 80°C erhitzt, nach Abkühlen auf 25°C wurde das Solvenz mit Hilfe einer Kanüle und eines Spritzenfilters mit einer Porengröße von 20 µm filtriert und dann das Silikagel 5 x mit 100 ml DMF gespült. Dann wurde eine Lösung von 2,45 g (= 11,42 mmol) von (S,S)-1,2-Diphenylethylendiamin in 150 ml entgastem DMF unter Rühren zu der Mischung gegeben und für 24 Stunden auf 80°C erhitzt. Nach Abkühlen wurde das Silikagel abfiltriert und 7 x mit 50 ml DMF und 7 x mit 50 ml Dichlormethan gewaschen. Dann wurde der immobilisierte Katalysator unter Hochvakuum bei 40°C für 12 Stunden getrocknet.
Ausbeute: 30,0 g.

### Beispiel 12

a) 3,35 g (S)-(6-cyclohexyloxy-6'-hydroxybiphenyl-2,2'-diyl)bis(diphenylphosphinoxid) (hergestellt analog zu Beispiel 6), 2,0 g N-(10-bromodecyl)-phthalimid und 3,2 g Kaliumcarbonat wurden zu 70 ml N,N-Dimethylformamid gegeben und die Mischung für 60 Stunden auf 80°C erhitzt. Nach Abkühlen wurden 100 ml Wasser zugegeben und die Mischung 3 x mit 30 ml Chloroform extrahiert. Die abgetrennten organischen Phasen wurden vereinigt, über Natriumsulfat getrocknet und mit Hilfe eines Rotationsverdampfers eingeengt. Analog zu der bekannten Gabrielsynthese von Aminen wurde das erhaltene Rohprodukt (4,8 g) durch Behandlung mit Hydrazinhydrat, Salzsäure und schließlich Natronlauge in (S)-[6-(10-aminodecyloxy)-6'-cyclohexyloxybiphenyl-2,2'-diyl]bis(diphenylphosphinoxid) überführt. Nach chromatographischer Reinigung (Silikagel Merck 60, Laufmittel: Ethylacetat/Methanol/Wasser 75:3:1,5), betrug die Ausbeute an reinem Produkt 3,2 g.
b) Das erhaltene reine Produkt (3,2 g) wurde in 100 ml Chloroform gelöst und die Lösung mit 0,6 g Triethylamin versetzt. Nach Abkühlen auf 0 bis 5°C wurde diese Lösung über einen Zeitraum von 20 Minuten mit Hilfe eines Tropftrichters mit einer Lösung von 0,4 g Methacryloylchlorid in 10 ml Chloroform versetzt. Dann wurde die Mischung über einen Zeitraum von 6 Stunden bei Raumtemperatur gerührt. Die Mischung wurde am Rotationsverdampfer eingeengt, anschließend mit 100 ml Chloroform versetzt und die Mischung mit 50 ml wässriger 2 N Salzsäure und 50 ml Wasser extrahiert. Die organische Phase wurde abgetrennt, über Natriumsulfat getrocknet und am Rotationsverdampfer eingeengt. Das Produkt wurde durch FlashChromatographie gereinigt.
   Ausbeute: 3,0 g (S)-[6-(10-N-Methacryloylamido-decyloxy)-6'-cyclohexyloxybiphenyl-2,2'-diyl]bis(diphenylphosphinoxid).

## Patentansprüche

1. Verfahren zur Herstellung von nicht-chiralen oder optisch aktiven Alkoholen, bei dem man eine Carbonylverbindung mit Wasserstoff in Gegenwart eines Katalysators, einer Base und gegebenenfalls eines Diamins umsetzt, **dadurch gekennzeichnet, dass** man als Katalysator einen trägergebundenen Ru(II)-Biphosphin-Diamin-Ru-Komplex-Katalysator der Formel (I) einsetzt
[Träger]-[gegebenenfalls Modifizierung]-[Bindungsgruppe]--[Bisphosphin 〉RuHal₂〈 Diamin] (I)
Hal = Cl oder Br.

2. Verfahren nach Anspruch 1, **dadurch gekennzeichnet, dass** es in Gegenwart der Verbindung mit der Formel (II), 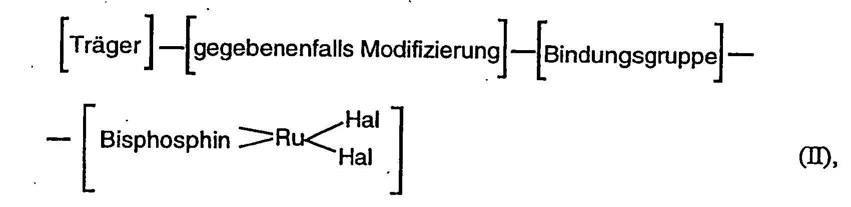 wobei
Hal Chlor oder Brom
bedeutet,
einer Base und eines Diamins durchgeführt wird, wobei sich der Katalysator mit der Formel (I) in situ bildet.

3. Verfahren nach Anspruch 1, **dadurch gekennzeichnet, dass** ein Katalysator verwendet wird, der sowohl einen chiral einheitlichen, trägergebundenen Bisphosphinliganden als auch einen chiral einheitlichen Diaminliganden entält.

4. Verfahren nach Anspruch 3, **dadurch gekennzeichnet, dass** der Katalysator einen atropisomeren Bisphosphinliganden enthält.

5. Ru(II)-Komplex-Katalysator mit der Formel (I)
[Träger]-[gegebenenfalls Modifizierung]-[Bindungsgruppe]--[Bisphosphin 〉RuHal₂〈 Diamin] (I),
wobei
Hal Chlor der Brom
bedeutet.

6. Ru(II)-Komplex nach Anspruch 5, **dadurch gekennzeichnet, dass** der Ru-Komplex durch Verknüpfung eines SH-Gruppen enthaltenden, anorganischen Trägers mit einem polymerisationsfähigen Bisphosphin(derivat) erhalten wurde.

7. Verbindungen der Formeln M¹, M², M³, M⁴, M⁵, M⁶, M⁷, M⁸, M⁹, M^{9'}, M¹⁰ und M^{10'} 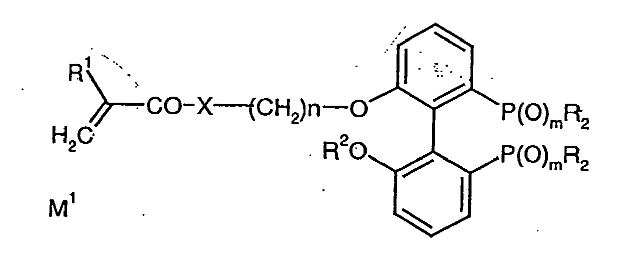 worin in der Formel M¹
R für Phenyl, 2- oder 3- oder 4-Methylphenyl, 3,5-Dimethylphenyl, 3,5-Dimethyl-4-methoxyphenyl, 3,5-Di-tert-butylphenyl oder Cyclohexyl,
R¹ steht für Wasserstoff oder Methyl,
X für O oder NH,
R² steht für Methyl, Ethyl, n- oder i-Propyl, n- oder i-Butyl, n-Pentyl, n-Hexyl, Cyclohexyl, n-Heptyl oder n-Octyl,
n steht für eine Zahl von 2 bis 12,
m steht für Null oder 1, vorzugsweise 1, 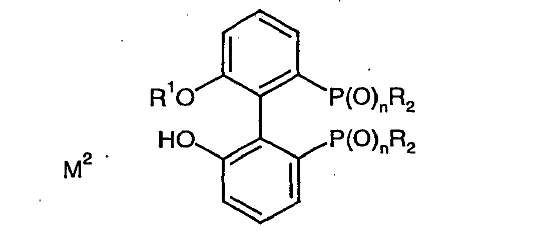 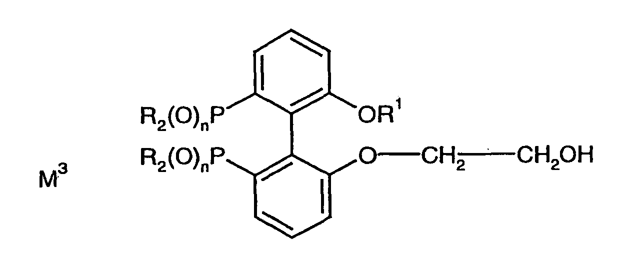 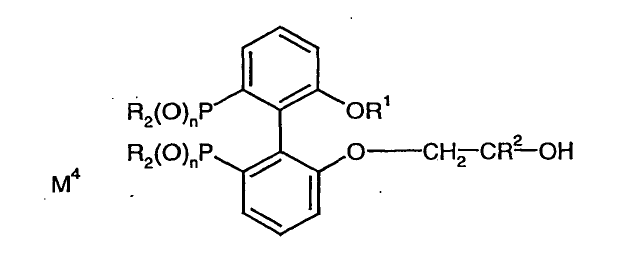 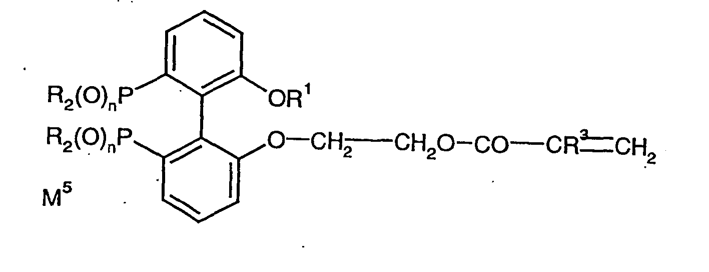 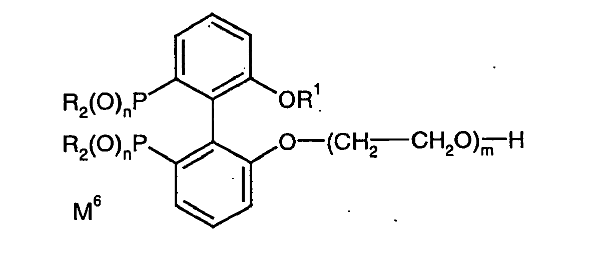 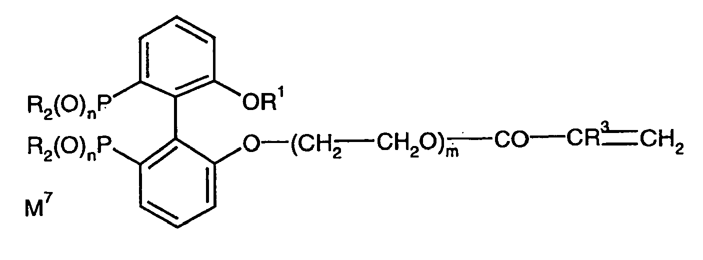 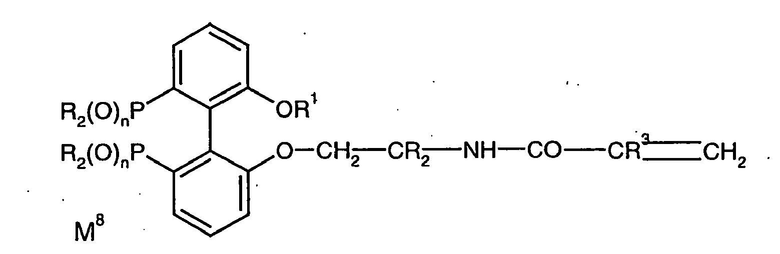 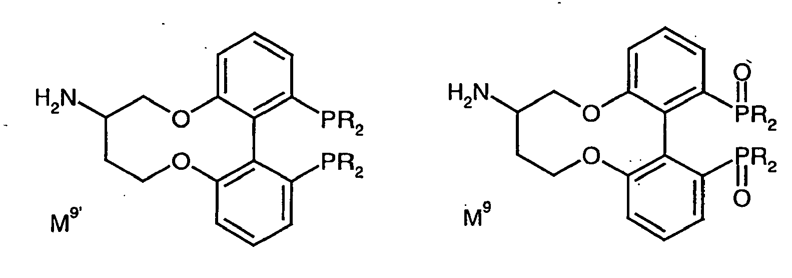 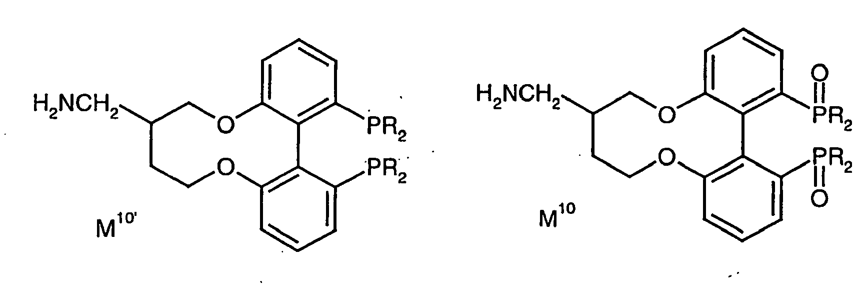 wobei jeweils
R für Phenyl, 2- oder 3- oder 4-Methylphenyl, 3,5-Dimethylphenyl, 3,5-Dimethyl-4-methoxyphenyl, 3,5-Di-tert-butylphenyl oder Cyclohexyl, steht und
R¹ und R² jeweils unabhängig voneinander für C₁- bis C₈-(Cyclo)Alkyl und
R³ für H oder CH₃ und
n für 1 oder Null und
m für 2 - 100 steht.

## Claims

1. Process for preparing achiral or optically active alcohols, in which a carbonyl compound is reacted with hydrogen in the presence of a catalyst, of a base and optionally of a diamine, **characterized in that** the catalyst used is a support-bound Ru(II)-biphosphine-diamine-Ru complex catalyst of the formula (I)
[support]-[optional modification]-[bonding group]--[bisphosphine 〉RuHal₂〈 diamine] (I)
Hal = Cl or Br.

2. Process according to Claim 1, **characterized in that** it is carried out in the presence of the compound of the formula (II) 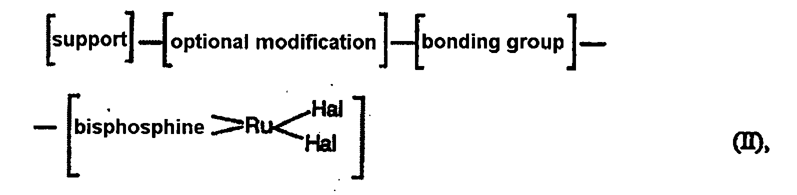 where
Hal is chlorine or bromine,
of a base and of a diamine, the catalyst of the formula (I) being formed in situ.

3. Process according to Claim 1, **characterized in that** a catalyst is used which contains both a chirally homogeneous, support-bound bisphosphine ligand and a chirally homogeneous diamine ligand.

4. Process according to Claim 3, **characterized in that** the catalyst contains an atropisomeric bisphosphine ligand.

5. Ru(II) complex catalyst of the formula (I)
[support]-[optional modifcation]-[bonding group]--[bisphosphine 〉RuHal₂〈 diamine] (I),
where
Hal is chlorine or bromine.

6. Ru(II) complex according to Claim 5, **characterized in that** the Ru complex has been obtained by joining an inorganic support containing SH groups to a polymerizable bisphosphine (derivative).

7. Compounds of the formulae M¹, M², M³, M⁴, M⁵, M⁶, M⁷, M⁸, M⁹, M^{9'} M¹⁰ and M^{10'} 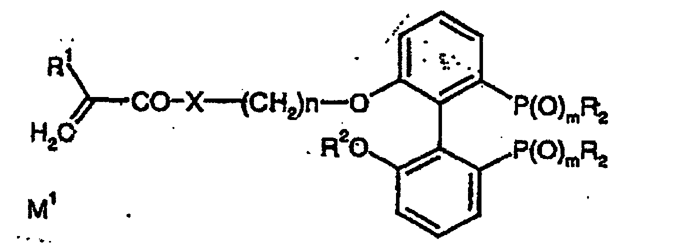 where, in the formula M¹,
R is phenyl, 2- or 3- or 4-methylphenyl, 3,5-dimethylphenyl, 3,5-dimethyl-4-methoxyphenyl, 3,5-di-tert-butylphenyl or cyclohexyl,
R¹ is hydrogen or methyl,
X is O or NH,
R² is methyl, ethyl, n- or i-propyl, n- or i-butyl, n-pentyl, n-hexyl, cyclohexyl, n-heptyl or n-octyl,
n is from 2 to 12,
m is zero or 1, preferably 1,

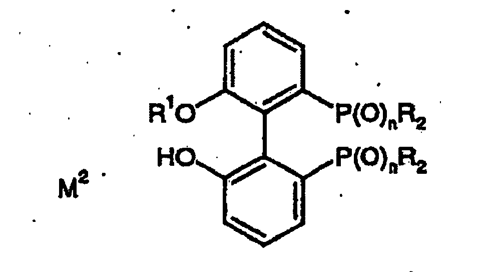

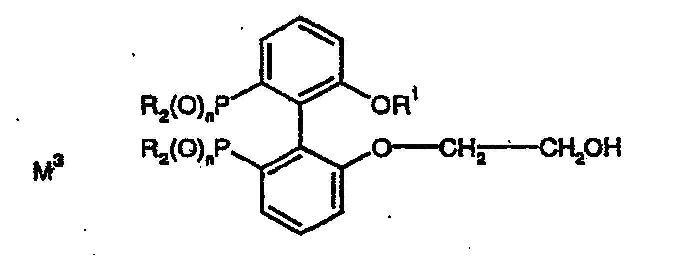

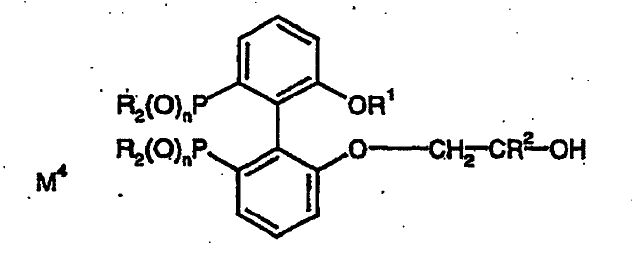

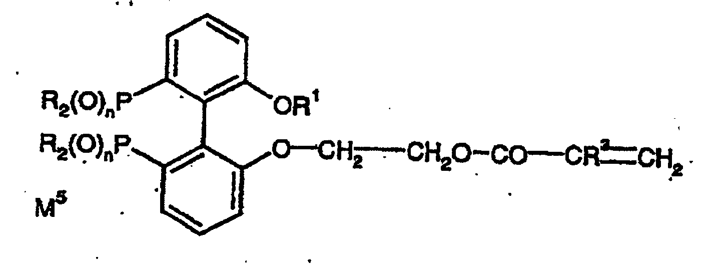

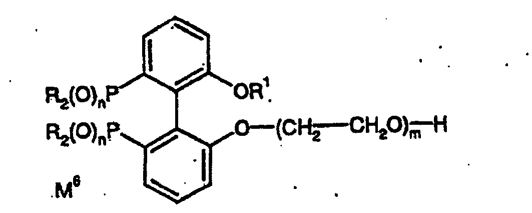

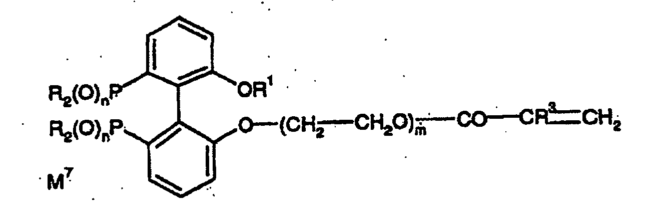

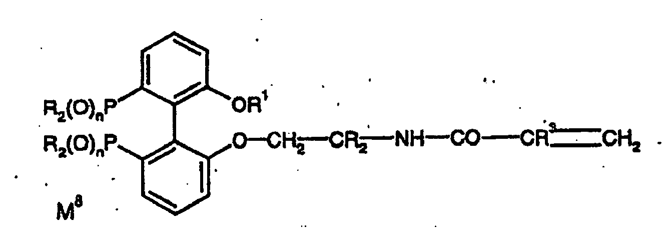

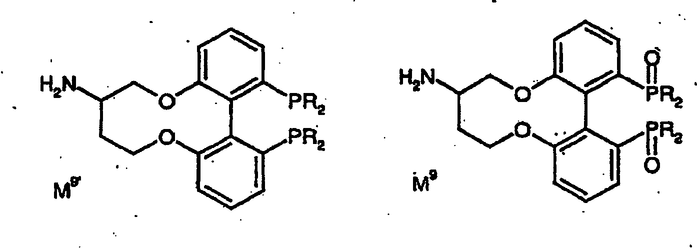

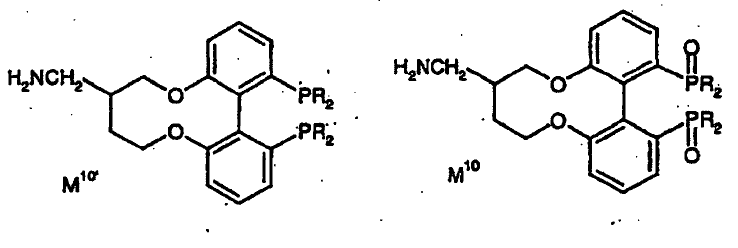

## Revendications

1. Procédé pour la préparation d'alcools non chiraux ou optiquement actifs, dans lequel on transforme un composé carbonyle avec de l'hydrogène en présence d'un catalyseur, d'une base et le cas échéant d'une diamine, **caractérisé en ce qu'**on utilise comme catalyseur un catalyseur complexe de Ru de type Ru(II)-diphosphinediamine lié à un support de formule (I)
[support]-[le cas échéant modification]-[groupe de liaison]-[diphosphine >RuHal₂<diamine] (I)
Hal = Cl ou Br.

2. Procédé selon la revendication 1, **caractérisé en ce qu'**il est réalisé en présence du composé de formule (II), 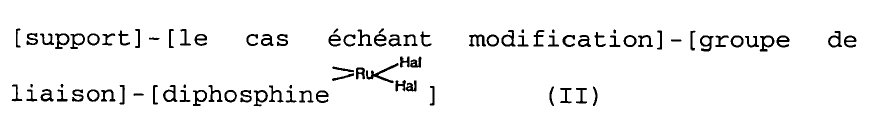 où
Hal signifie chlore ou brome
d'une base et d'une diamine, le catalyseur de formule (I) se formant in situ.

3. Procédé selon la revendication 1, **caractérisé en ce qu'**on utilise un catalyseur qui contient tant un ligand de type diphosphine chiralement uniforme, lié à un support qu'un ligand de type diamine chiralement uniforme.

4. Procédé selon la revendication 3, **caractérisé en ce que** le catalyseur contient un ligand de type diphosphine atropisomère.

5. Catalyseur complexe de Ru(II) de formule (I)
[support]-[le cas échéant modification]-[groupe de liaison]--[diphosphine >RuHal₂<diamine] (I)
où
Hal signifie chlore ou brome.

6. Complexe de Ru(II) selon la revendication 5,
**caractérisé en ce que** le complexe de Ru est obtenu par liaison d'un support inorganique contenant des groupes SH avec une diphosphine (ou un dérivé de celle-ci) apte à la polymérisation.

7. Composés des formules M¹, M², M³, M⁴, M⁵, M⁶, M⁷, M⁸, M⁹, M^{9'}, M¹⁰ et M^{10'} 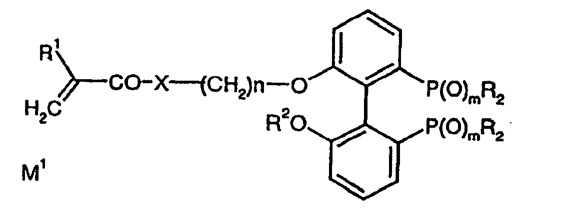 où, dans la formule M¹
R représente phényle, 2-méthylphényle ou 3-méthylphényle ou 4-méthylphényle, 3,5-diméthylphényle, 3,5-diméthyl-4-méthoxyphényle, 3,5-di-tert-butylphényle ou cyclohexyle,
R¹ représente hydrogène ou méthyle,
X représente O ou NH,
R² représente méthyle, éthyle, n-propyle ou i-propyle, n-butyle ou i-butyle, n-pentyle, n-hexyle, cyclohexyle, n-heptyle ou n-octyle,
n représente un nombre de 2 à 12,
m représente zéro ou 1, de préférence 1, 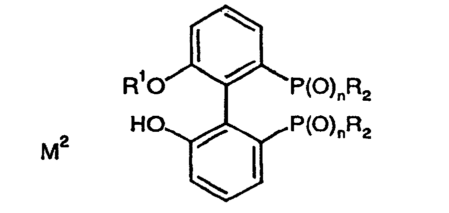 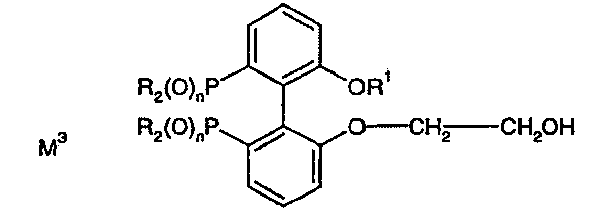 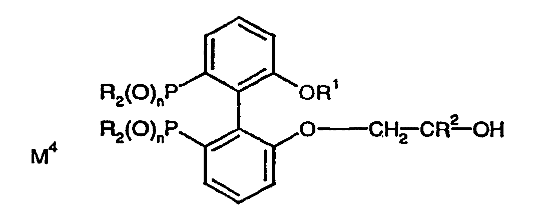 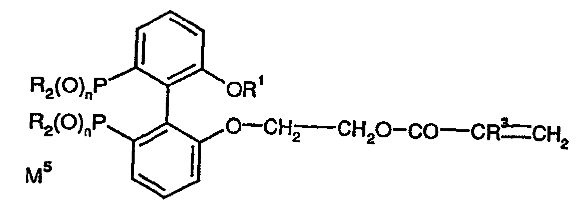 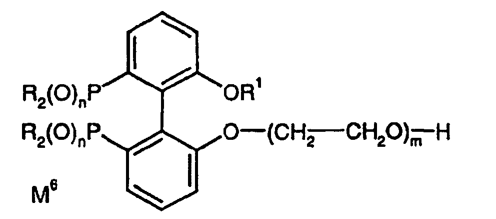 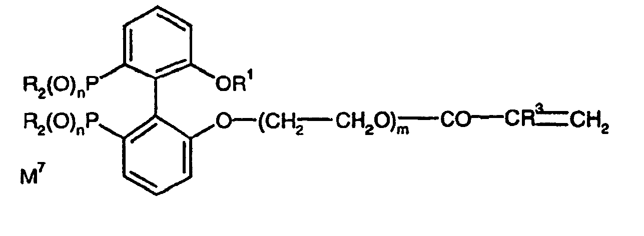 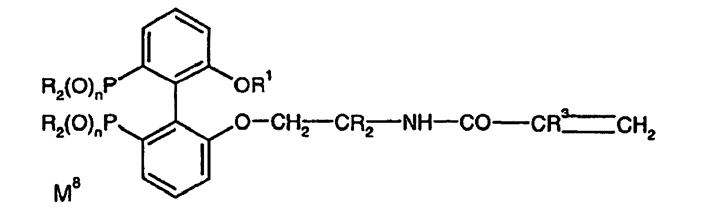 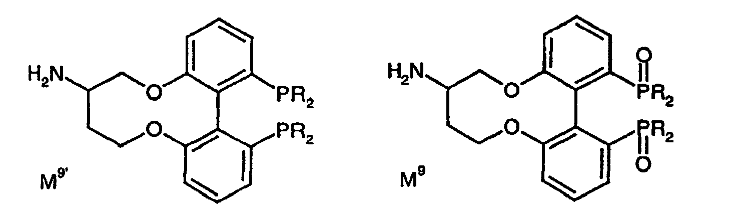 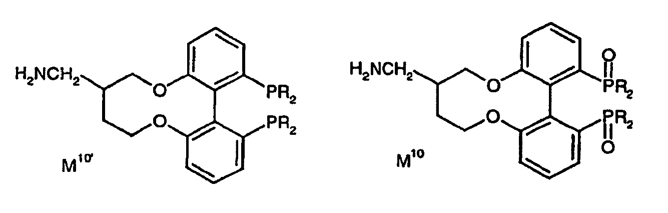 où, à chaque fois
R représente phényle, 2-méthylphényle ou 3-méthylphényle ou 4-méthylphényle, 3,5-diméthylphényle, 3,5-diméthyl-4-méthoxyphényle, 3,5-di-tert-butylphényle ou cyclohexyle, et
R¹ et R² représentent, à chaque fois indépendamment l'un de l'autre, C₁-C₈-(cyclo)alkyle et
R³ représente H ou CH₃ et
n vaut 1 ou zéro et
m vaut 2-100.
